# EUROPEAN PATENT SPECIFICATION

(11) **EP 2 679 270 B1**
(45) Date of publication and mention of the grant of the patent: **09.11.2016**
(21) Application number: 12749490.4
(22) Date of filing: 23.02.2012
(51) Int. Cl.: A61M 37/00, A61K 38/26, A61K 9/00, A61K 47/10

(54) **GLP-1 ANALOGUE COMPOSITION FOR MICRONEEDLE DEVICES**
GLP-1-ANALOG-ZUSAMMENSETZUNG FÜR MIKRONADELVORRICHTUNGEN
COMPOSITION D'ANALOGUE DE GLP-1 POUR DES DISPOSITIFS DE MICRO AIGUILLE

(30) Priority: 24.02.2011 JP 2011038695
(43) Date of publication of application: 01.01.2014
(73) Proprietor: Hisamitsu Pharmaceutical Co., Inc., Tosu-shi, Saga 841-0017 (JP)
(72) Inventor: UMEMOTO, Takuya, Tsukuba-shi Ibaraki 305-0856 (JP); MATSUDO, Toshiyuki, Tsukuba-shi Ibaraki 305-0856 (JP); TOKUMOTO, Seiji, Tsukuba-shi Ibaraki 305-0856 (JP)
(74) Representative: Grünecker Patent- und Rechtsanwälte PartG mbB
(86) International application number: PCT/JP2012/054465
(87) International publication number: WO 2012/115208

(56) References cited:
- EP-A1- 2 540 337
- WO-A1-2009/029847
- WO-A1-2011/007722
- WO-A2-2006/138719
- WO-A2-2010/092163
- JP-A- 2010 538 017
- US-A1- 2006 030 838
- CHRISTENSEN M ET AL: "Lixisenatide, a novel GLP-1 receptor agonist for the treatment of type 2 diabetes mellitus", I DRUGS: THE INVESTIGATIONAL DRUGS JOURNAL, CURRENT DRUGS LTD, GB, vol. 12, no. 8, 1 August 2009 (2009-08-01) , pages 503-513, XP009163477, ISSN: 2040-3410

## Description

### Technical Field

The present invention relates to a GLP-1 analogue composition for a microneedle device.

### Background Art

Recently, incretin-related drugs have been attracting attention as therapeutic drugs for type II diabetes, and the various GLP-1 analogue preparations have been actively developed and the use of these preparations has been approved. GLP stands for glucagon-like peptide, which is originally an incretin hormone secreted from the human digestive tract mucosa. The analogue preparations of glucagon-like peptide 1 (GLP-1) are agonists of the GLP-1 receptor, and in the living body, GLP-1 is secreted from the small intestine, and in the periphery, it promotes insulin secretion in the pancreatic β cells while suppressing glucagon secretion in the pancreatic α cells, whereas in the center, it acts as a food intake-suppressing hormone. By virtue of these actions, GLP-1 accelerates insulin secretion only when the concentration at which insulin secretion is stimulated has been reached, that is, in a glucose concentration-dependent manner, thereby effectively reducing blood glucose. Due to these characteristics, GLP-1 is considered to be a candidate drug suitable for the treatment of type II diabetes.

However, because GLP-1 secreted in the living body is rapidly degraded by dipeptidyl peptidase-4 (DPP-4), its elimination half-life (t_{1/2}) is very short (less than 1.5 minutes after intravenous administration), and because its duration of action is short, it is not suitable as a therapeutic drug for diabetes, and in light of this, drugs such as various kinds of GLP-1 analogues have been developed (Non Patent Literatures 1 and 2).

Lixisenatide is a human GLP-1 analogue preparation for the treatment of type II diabetes. Lixisenatide is made resistant to degradation by DPP-4 by replacing alanine in GLP-1 by glycine, and is known as a derivative in which six lysine residues are added to the C-terminus based on exendin-4 (1 to 39). Since affinity to arginine is increased by adding lysine to the C-terminus, lixisenatide is designed to maintain its blood concentration. Further, lixisenatide is perceived as superior to existing drugs not only for maintaining the insulin synthetic capacity of pancreas and retarding progression of disease and occurrence of complications, but also in terms of normalization of long-term blood glucose, suppression of postprandial hypoglycemia, suppression of body weight gain, and the like.

GLP-1 analogue preparations are peptide medicines and are rapidly digested when orally administered, and in light of this, GLP-1 analogue preparations are developed as subcutaneous injection preparations. However, because the above drugs are drugs that require frequent administration, namely once per day to once per week, when administration is given by injection, burden (pain) imposed on the patients tends to be high. In view of the foregoing, there is a strong demand for the development of a more convenient administration method that can prevent pain at the time of administration.

Meanwhile, as a device for improving transdermal absorption of drugs, a microneedle device is known. With regard to the microneedles to be disposed on a microneedle device, various sizes and forms have been proposed for the purpose of piercing the stratum corneum, which is the outermost skin layer. Administration by a microneedle device is expected as a non-invasive administration method involving no pain at the time of administration (for example, Patent Literature 1).

Various methods as the method of application of a drug when utilizing a microneedle device have been also proposed. For example, coating the microneedle surface with a drug, disposing a groove or hollow part in microneedles through which a drug or biological component is allowed to penetrate, and mixing a drug with the microneedle itself are known. There is a disclosure that the substance that is mixed together at the time of coating with a drug preferably contains sugars, and in particular stabilizing sugars that form a glass (amorphous solid material) such as lactose, raffinose, trehalose, or sucrose (Patent Literature 2).

Patent Literature 3 discloses a device and method for transdermal delivery of a biologically active drug, comprising a delivery system having a microprojection member. In one aspect of it, there is a disclosure that the biocompatible coating formulation applied to a microprojection member contains at least one kind of non-aqueous solvent, such as ethanol, isopropanol, methanol, propanol, butanol, propylene glycol, dimethylsulfoxide, glycerin, N,N-dimethylformamide, and polyethylene glycol 400, and preferably, the non-aqueous solvent is present in the coating formulation in the range of approximately 1 to 50% by mass of the coating formulation. It is also disclosed that the viscosity of this coating formulation is 3 to approximately 500 centipoise (cps).

Various methods have been disclosed also for the method of coating microneedles. For example, Patent Literature 4 describes a method of coating by immersion (immersion coating).

Also, for example, Patent Literature 5 discloses administration of a GLP-1 or GLP-2 analogue by microneedles coated therewith.

### Citation List

### Patent Literature

Patent Literature 1: Japanese Unexamined Patent Application Publication of International Patent Application No. 2001-506904
Patent Literature 2: Japanese Unexamined Patent Application Publication of International Patent Application No. 2004-504120
Patent Literature 3: Japanese Unexamined Patent Application Publication of International Patent Application No. 2007-536988
Patent Literature 4: U.S. Unexamined Patent Application Publication No. 2008/0213461
Patent Literature 5: U.S. Unexamined Patent Application Publication No. 2009/0136554

### Non Patent Literature

Non Patent Literature 1: Yusuke Hada and two others, "Tonyobyo chiryoyaku: incretin kanren shinyaku gaisetu", Iyaku Journal, Iyaku Journal Co., Ltd., 2010, Vol. 46 S-1, pp. 223 to 228
Non Patent Literature 2: Junichiro Miyagawa and one other, "Incretin mimetics: GLP-1 receptor agonists", Igaku no ayumi, Ishiyaku Pub., Inc., 2009, Vol. 231, No. 7, pp. 767 to 772

### Summary of Invention

### Technical Problem

However, it has been revealed that in a case in which a GLP-1 analogue is administered by microneedles coated therewith, the coating composition described in Patent Literature 5 is associated with such problems that a drug is not adequately dissolved in the coating composition, and the coating composition often causes dripping after microneedles are coated therewith due to its low viscosity.

Further, the present inventors found that in a case in which a GLP-1 analogue is administered, from the viewpoint of long-term stability of the drug and favorable coating properties on the tip of the needle (particularly, resistance to dripping from the tip of the needle), water is advantageous, whereas from the viewpoint of easiness in obtaining suitable liquid properties, a polyhydric alcohol is advantageous as a solvent in a coating agent.

Further, there have been problems that when water was selected, a viscosity suitable for coating microneedles was difficult to obtain, and the present inventors also found at the same time that a polymeric carrier was effective to solve the above problem. Further, there have also been cases that when a polyhydric alcohol was selected, a viscosity suitable for coating microneedles was difficult to obtain under certain circumstances (for example, when the drug concentration was set low), and the present inventors also found that particularly when the drug concentration in a formulation was low, improving the viscosity by the addition of a polymeric carrier was effective.

An object of the present invention is to provide a GLP-1 analogue composition for a microneedle device having such a high viscosity that enables to suppress dripping after coating.

### Solution to Problem

The present invention provides a GLP-1 analogue composition for a microneedle device, including a GLP-1 analogue and water as a solvent.

The aforementioned GLP-1 analogue composition for a microneedle device is high in viscosity, and thus the aforementioned GLP-1 analogue composition adhering to microneedles can be controlled to a desired thickness.

The aforementioned GLP-1 analogue is preferably lixisenatide.

The GLP-1 analogue composition further includes pullulan as a polymeric carrier.

The present invention further provides the aforementioned GLP-1 analogue composition for a microneedle device adhering to microneedles.

### Advantageous Effects of Invention

The GLP-1 analogue composition for a microneedle device according to the present invention is high in viscosity. Therefore, when microneedles are coated with the aforementioned GLP-1 analogue composition, dripping on the microneedles can be suppressed and the GLP-1 analogue content can be maintained at a constant level, and the adhering GLP-1 analogue composition can be controlled to a desired thickness. Further, with regard to the microneedle device having the GLP-1 analogue composition for a microneedle device according to the present invention, the releasing properties of the GLP-1 analogue is favorable when it is administered.

### Brief Description of Drawings

Figure 1 is a perspective view showing one embodiment of a microneedle device.
Figure 2 is a cross-sectional view of Figure 1 taken along the line II - II.
Figures 3(a) to (c) are diagrams showing one embodiment of a method for producing a microneedle device.
Figure 4 is a photograph of microneedles with the GLP-analogue composition adhering thereto.
Figure 5 is a photograph of microneedles with the GLP-analogue composition adhering thereto.
Figure 6 is a photograph of microneedles with the GLP-analogue composition adhering thereto.
Figure 7 is a graph showing the changes in the blood lixisenatide concentration with time when lixisenatide was administered by a microneedle device or by the subcutaneous administration.
Figure 8 is a graph showing the lixisenatide content in a microneedle device after it is left to stand as a relative value with respect to the initial value.

### Description of Embodiments

Hereinbelow, preferable embodiments will be explained with reference to drawings. It is to be noted that in the explanation of the drawings, the same symbols are assigned to the same elements and redundant explanation will be omitted. Also, in the drawings, some parts are exaggeratedly drawn to make understanding easy, and thus the size and ratio are not necessarily consistent with the description.

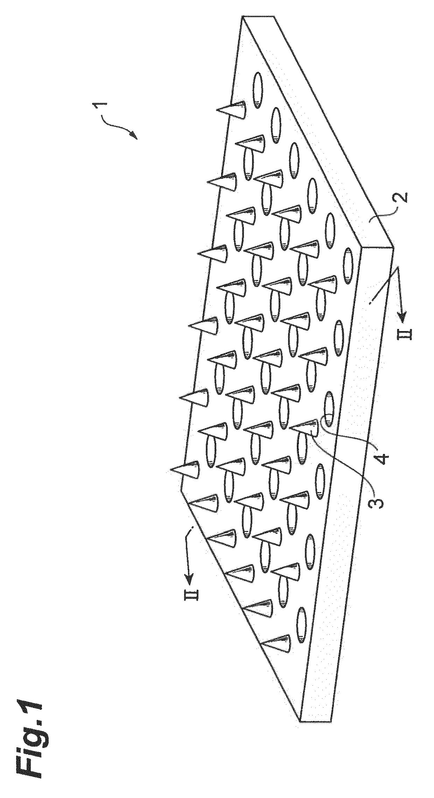

Figure 1 is a perspective view showing one embodiment of a microneedle device obtained by using a GLP-1 analogue composition of the present invention. A microneedle device 1 shown in Figure 1 has a microneedle substrate 2 and a plurality of microneedles 3 that are two-dimensionally arranged on the microneedle substrate 2.

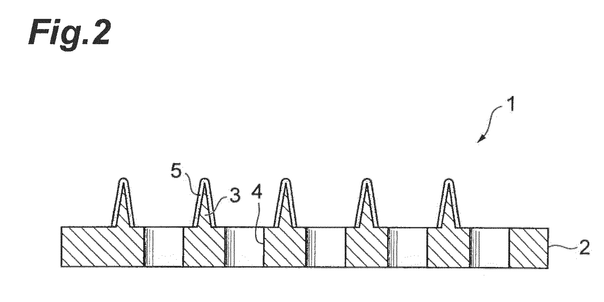

Figure 2 is a cross-sectional view of Figure 1 taken along the line II - II. As shown in Figure 2, the microneedle device 1 has the microneedle substrate 2, the microneedles 3 disposed on the microneedle substrate 2, and a GLP-1 analogue composition 5 adhering to the microneedles 3 and/or the substrate. As will be described later, the GLP-1 analogue composition 5 adhering to the microneedles includes (A) a "GLP-1 analogue" and (B) "at least one kind of solvent selected from the group consisting of water, glycerin, propylene glycol, ethylene glycol, 1,3-butylene glycol, and polyethylene glycol."

The microneedle substrate 2 is a foundation to support the microneedles 3. The form of the microneedle substrate 2 is not particularly limited, and for example, on the microneedle substrate 2, a plurality of through holes 4 may be formed so that they are two-dimensionally arranged. The microneedles 3 and the through holes 4 are alternately arranged in the direction of a diagonal of the microneedle substrate 2. By virtue of the through holes 4, the GLP-1 analogue can be administered from the back of the microneedle substrate 2. However, a substrate lacking such a through hole may also be used. The area of the microneedle substrate 2 is 0.5 to 10 cm², preferably 1 to 5 cm², and more preferably 1 to 3 cm². A substrate of a desired size may be configured by connecting several pieces of the microneedle substrates 2.

The microneedles 3 each have a minute structure, and the height (length) thereof is preferably 50 to 600 µm. At this point, the reason for setting the length of the microneedles 3 at 50 µm or more is to ensure transdermal administration of a GLP-1 analogue, and the reason for setting the length at 600 µm or less is to avoid that the microneedles contact nerves so as to securely reduce the possibility of pain and securely avoid the possibility of bleeding. Also, when the length of the microneedles 3 is 500 µm or less, the amount of a GLP-1 analogue to enter the skin can be efficiently administered, and in certain cases, administrating without piercing the skin is also possible. The length of the microneedles 3 is particularly preferably 300 to 500 µm.

At this point, a microneedle refers to a projecting structure including, in a broad sense, a needle shape or a structure including a needle shape. However, the microneedle is not limited to a structure of a needle shape having a tapered tip but also includes a structure lacking a tapered tip. When the microneedles 3 each have a conical structure, the diameter of the basal surface thereof is approximately 50 to 200 µm. Although the microneedles 3 are each in a conical shape according to the present embodiment, microneedles in a polygonal pyramid shape such as a square pyramid or in other shapes may also be used.

The microneedles 3 are each typically disposed spaced apart so that a density of approximately one to 10 needles per millimeter (mm) is provided in a row of the needles. Generally, adjacent rows are spaced apart from each other by a distance substantially equal to the space between the needles in a row, and the microneedles 3 have a needle density of 100 to 10000 needles per cm². When a needle density of 100 needles or more is achieved, the microneedles can efficiently pierce the skin. Meanwhile, a needle density of more than 10000 needles makes it difficult to maintain the strength of the microneedles 3. The density of the microneedles 3 is preferably 200 to 5000 needles, more preferably 300 to 2000 needles, and particularly preferably 400 to 850 needles.

Examples of a material of the microneedle substrate 2 or the microneedles 3 include silicon, silicon dioxide, ceramics, metals (such as stainless steel, titanium, nickel, molybdenum, chromium, and cobalt), and synthetic or natural resin materials. In consideration of the antigenicity of the microneedles and the unit price of the material, a biodegradable polymer such as polylactic acid, polyglycolide, polylactic acid-co-polyglycolide, pullulan, polycaprolactone, polyurethane, and polyanhydride, and a synthetic or natural resin material such as polycarbonate, polymethacrylic acid, ethylenevinyl acetate, polytetrafluoroethylene, and polyoxymethylene, which are non-biodegradable polymers, are particularly preferable. Further, polysaccharides such as hyaluronic acid, sodium hyaluronate, pullulan, dextran, dextrin, and chondroitin sulfate are also suitable.

Examples of a production method of the microneedle substrate 2 or the microneedles 3 include wet etching process or dry etching process using a silicon substrate, precision machining using metals or resins (such as electric discharge method, laser processing, dicing processing, hot embossing process, and injection mold processing), and machinery cutting. By these processing methods, a needle part and a support part are integrally molded. Examples of a method for hollowing the needle part include a method in which a secondary processing such as laser processing is applied after producing the needle part.

Examples of a method for applying the aforementioned GLP-1 analogue composition onto the microneedles and/or substrate include spray coating and immersion coating, and the method of application is preferably immersion coating.

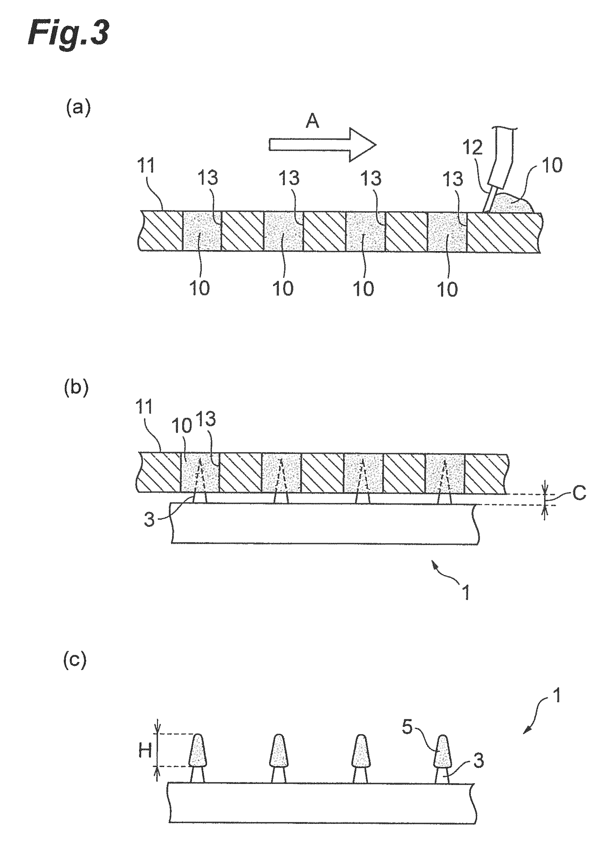

(a), (b) and (c) of Figures 3 are diagrams showing one embodiment of a method for producing the microneedle device 1. According to this method, first of all, as shown in Figure 3(a), a GLP-1 analogue composition 10 is swept in the direction of the arrow A by a spatula 12 on a mask plate 11. By doing so, openings 13 are filled with the GLP-1 analogue composition. Subsequently, as shown in Figure 3(b), the microneedles 3 are inserted into the openings 13 on the mask plate 11. Subsequently, as shown in Figure 3(c), the microneedles 3 are pulled out from the openings 13 on the mask plate 11. By doing so, the GLP-1 analogue composition 10 adheres to the microneedles 3 (on the microneedles and/or substrate). Subsequently, the GLP-1 analogue composition on the microneedles is dried by a known method such as air drying, vacuum drying, freeze drying, or a combination of these methods. By the above process, a solid GLP-1 analogue composition 10 more strongly adheres to the microneedles 3 as a GLP-1 analogue composition 5 adhering to the microneedles 3. That is, the solid GLP-1 analogue composition 10 is firmly fixed to the microneedles 3. A microneedle device is produced as described above. It is to be noted that the water content in the GLP-1 analogue composition that has adhered to the microneedles by drying the GLP-1 analogue composition is normally 7% by mass or less, preferably 5% by mass or less or 3% by mass or less based on the total amount of the composition.

The height H of the GLP-1 analogue composition adhering to the microneedles 3 is adjusted by clearance (gap) C as shown in Figure 3(b). This clearance C is defined as a distance between the basal surface of the microneedles and the surface of the mask (substrate thickness is not involved), and is set according to a tension of the mask plate 11 and the length of the microneedles 3. The range of the distance of clearance C is preferably 0 to 500 µm. When a distance of clearance C is 0, it means that the GLP-1 analogue composition is applied to the entire microneedles 3. Although the height H of the GLP-1 analogue composition adhering to the microneedles 3 varies depending on the height h of the microneedles 3, the height H may be set at 0 to 500 µm, normally at 10 to 500 µm, and preferably approximately at 30 to 300 µm.

The thickness of the GLP-1 analogue composition 5 adhering to the microneedles 3 is less than 50 µm, preferably less than 40 µm, and more preferably 1 to 30 µm. Generally, the thickness of the GLP-1 analogue composition adhering to the microneedles refers to an average thickness as measured over the surface of the microneedles 3 after drying. Generally, the thickness of the GLP-1 analogue composition adhering to the microneedles can be increased by applying a plurality of films of the GLP-1 analogue composition, namely, by repeatedly performing the step of adhesion on the microneedles 3 after the GLP-1 analogue composition has been already adhered thereto.

When the GLP-1 analogue composition is allowed to adhere to the microneedles 3, temperature and humidity in an installation environment of an apparatus are preferably controlled at a constant level. Also, when necessary, the environment may be filled with a solvent used for the GLP-1 analogue composition, which is a component (B) to be described later. By doing so, evaporation of the solvent in the GLP-1 analogue composition can be prevented as much as possible.

The GLP-1 analogue composition for a microneedle device contains (A) a "GLP-1 analogue" and (B) "water as solvent. " The "GLP-1 analogue" refers to a derivative of a peptide for glucagon, which has an agonist action of GLP-1 receptor. Examples of the GLP-1 analogue include exenatide, liraglutide, lixisenatide, albiglutide, and taspoglutide. Among them, lixisenatide is preferable.

These drugs may be used singly or in combination of two or more kinds thereof, and they encompass a drug in the form of either an inorganic acid salt or an organic acid salt, as long as they are pharmaceutically acceptable salts. The content of (A) the "GLP-1 analogue" in the composition is preferably 0.1 to 80% by mass, more preferably 1 to 70% by mass, and further preferably 5 to 60% by mass.

(B) the solvent is water (purified water) and may volatilize when the GLP-1 analogue composition is adhered thereto, making adhesion difficult. Therefore, the boiling point of the aforementioned solvent is 100°C or more.

When the solubility of a GLP-1 analogue is low and a coating carrier is required, the solvent is water. A combination of water and these GLP-1 analogues is superior in coating properties, blood concentration profile, and storage stability.

Since (B) the "water as solvent" has a high solubility or dispersibility for a GLP-1 analogue, a microneedle device in which the content of the adhering GLP-1 analogue composition is uniform can be obtained. The blending ratio of the component (A) to the component (B) (A:B) in the composition is preferably 20:80 to 80:20, more preferably 40:60 to 80:20, and particularly preferably 40:60 to 70:30 by mass.

The component (B) is water, the GLP-1 analogue composition further includes pullulan as a polymeric carrier (tackifier). By virtue of a combination of water and a polymeric carrier, a viscosity of a composition suitable for coating is particularly easily obtained.

As the polymeric carrier, a carrier highly compatible (having the property of being uniformly mixed) with a GLP-1 analogue is preferable. In this respect, the polymeric carrier is pullulan.

The content of the polymeric carrier in the GLP-1 analogue composition is preferably 1 to 70% by mass, more preferably 1 to 40% by mass, and further preferably 3 to 25% by mass. The polymeric carrier preferably has a viscosity of approximately 100 to 100000 cps at room temperature (25°C) so as to attain such a degree of viscosity that does not cause dripping. A more preferable viscosity of the polymeric carrier is 500 to 60000 cps. For the viscosity being within the above range, it becomes possible to apply a desired amount of a coating solution at once without depending on the material of the microneedles. Also, generally, there is a tendency that the higher the viscosity, the larger the amount of the GLP-1 analogue composition.

In addition, to the GLP-1 analogue composition, propylene carbonate, crotamiton, 1-menthol, peppermint oil, limonene, diisopropyl adipate, and the like may be added as a solubilizing aid or absorption promoter, and methyl salicylate, glycol salicylate, 1-menthol, thymol, peppermint oil, nonylic acid vanillylamide, chili pepper extract, and the like may be added as an efficacy supplement as needed.

To the GLP-1 analogue composition, a compound such as a stabilizer, an antioxidant, an emulsifier, a surfactant, and salts may be added as needed. In the present invention, the surfactant may be either a nonionic surfactant or an ionic surfactant (cationic, anionic, and amphoteric); however, from the safety aspect, a nonionic surfactant, which is normally used for a pharmaceutical base, is desirable. Examples of these compounds include sugar alcohol fatty acid ester such as sucrose fatty acid ester, sorbitan fatty acid ester, glycerin fatty acid ester, polyglycerin fatty acid ester, propylene glycol fatty acid ester, polyoxyethylene sorbitan fatty acid ester, polyoxyethylene glycerin fatty acid ester, polyethylene glycol fatty acid ester, polyoxyethylene castor oil, and polyoxyethylene hydrogenated castor oil.

Other known pharmaceutical aids may be added to the GLP-1 analogue composition as long as they do not drastically impair the effect of improving the solubility and viscosity of the GLP-1 analogue composition as well as the nature and physical properties of the GLP-1 analogue composition adhering to the dried microneedles.

The GLP-1 analogue composition must have a certain degree of viscosity so as not to cause dripping on the microneedles after applying. Specifically, the GLP-1 analogue composition desirably has a viscosity of approximately 100 to 100000 cps. A more preferable viscosity of the GLP-1 analogue composition is 100 to 60000 cps. For the viscosity being within the above range, it becomes possible to allow a desired amount of the GLP-1 analogue composition to adhere at once without depending on the material of the microneedles 3. Generally, there is a tendency that the higher the viscosity, the larger the amount of the GLP-1 analogue composition that can be adhered to the microneedles, and when the viscosity is less than 100 cps, there is a possibility that it becomes difficult to allow a minimum amount of GLP-1 analogue to adhere to the microneedles 3. However, unexpectedly, conversely, when the viscosity is 45000 cps or more, the content of a GLP-1 analogue in the resulting GLP-1 analogue composition 5 adhering to the microneedles starts to decrease. From these characteristics, there is a tendency that when the viscosity of the GLP-1 analogue composition is 45000 cps or more, it becomes difficult to increase the content of a GLP-1 analogue in the GLP-1 analogue composition 5 adhering to the microneedles to a sufficient level even by increasing the amount of the GLP-1 analogue used. Accordingly, the viscosity of the GLP-1 analogue composition is particularly preferably 600 to 45000 cps.

### Examples

Hereinbelow, the present invention will be more specifically described by providing Examples of the present invention; however, the present invention is not limited to these Examples and can be modified in various ways within a range that does not deviate from the technical idea of the present invention.

### <Solubility test and viscosity measurement for GLP-1 analogues>

### (Reference examples 1 to 6)

In tubes, lixisenatide and solvents were mixed at content ratios shown in Table 1. The liquid mixtures thus produced were stirred with a stirrer (1500 rpm, 12 hours, 25°C), and the solubility of the polymeric compounds was visually evaluated based on the following criteria. The evaluation results are shown in Table 1.
A: Completely dissolved.
B: Partially dissolved.
C: Not dissolved.

The viscosity of the liquid mixtures thus produced was evaluated based on the following criteria as the impression at the time of mixing.
a: Suitable for coating.
b: Not suitable for coating.

**[Table 1]**

| | Solvent | Solvent content (% by mass) | Lixisenatide content (% by mass) | Solubility | Viscosity |
|---|---|---|---|---|---|
| Example 1 | Glycerin | 62.5 | 37.5 | A | a |
| Example 2 | Propylene glycol | 50.0 | 50.0 | A | a |
| Example 3 | Ethylene glycol | 50.0 | 50.0 | A | a |
| Example 4 | Macrogol 200 | 50.0 | 50.0 | A | a |
| Example 5 | Macrogol 400 | 50.0 | 50.0 | A | a (Hard) |
| Example 6 | 1,3-Butylene glycol | 50.0 | 50.0 | A | a |

Macrogol 200 and Macrogol 400 in the Table each refer to polyethylene glycol of which molecular weight is 200 and 400, respectively.

### (Example 7)

Using water as a solvent and further adding a polymeric carrier, the components were mixed at content ratios shown in Table 2. The compatibility and viscosity of the resulting mixtures were evaluated in a similar manner to Examples 1 to 6. The evaluation results are shown in Table 2.

**[Table 2]**

| Polymeric carrier | Polymeric carrier content (% by mass) | Lixisenatide content (% by mass) | Water content (% by mass) | Solubility | Viscosity |
|---|---|---|---|---|---|
| Pullulan | 12.5 | 37.5 | 50.0 | A | a |
| PEG400 | 12.5 | 37.5 | 50.0 | A | b |
| PEG400 | 25.0 | 37.5 | 37.5 | A | b |
| PEG4000 | 12.5 | 37.5 | 50.0 | A | b |
| PEG4000 | 25.0 | 37.5 | 37.5 | A | b |
| HPC-M | 3.1 | 37.5 | 59.4 | A | a |
| HPC-M | 12.5 | 37.5 | 50.0 | B | b |
| CMC (250000) | 3.1 | 37.5 | 59.4 | A | b (Gel) |
| CMC (250000) | 12.5 | 37.5 | 50.0 | B | b |
| Dx-40 | 12.5 | 37.5 | 50.0 | A | a |
| Dx-40 | 25.0 | 37.5 | 37.5 | A | a (Hard) |
| Dx-70 | 12.5 | 37.5 | 50.0 | A | a |
| Dx-70 | 25.0 | 37.5 | 37.5 | A | a (Hard) |
| Na hyaluronate | 3.1 | 37.5 | 59.4 | A | b (Gel) |
| Na hyaluronate | 12.5 | 37.5 | 50.0 | B | b |

In the table, PEG400 and PEG4000 each refer to polyethylene glycol of which weight average molecular weight is 400 and 4,000, respectively; Dx40 and Dx70 each refer to dextran of which weight average molecular weight is 40,000 and 70,000, respectively; HPC-M refers to hydroxypropylcellulose of which weight average molecular weight is 110,000 to 150,000; and CMC (250000) refers to carboxymethylcellulose sodium of which weight average molecular weight is 250,000.

In Examples 1 to 6, excellent solubility and viscosity were exhibited. Also in Example 7, there were some cases in which excellent solubility and viscosity were exhibited. In Example 7, the thickening effect of the polymeric carrier added was not produced in some cases; however, even in those cases, it is presumably possible to obtain the thickening effect by increasing the amount of lixisenatide or a polymeric carrier added.

### <Coating of microneedles with lixisenatide>

### (Examples 8 to 10)

In tubes, lixisenatide and solvents (and polymeric carriers) were mixed at mass ratios shown in Table 3, whereby GLP-1 analogue compositions were obtained. Example 8 is a composition with an aqueous formulation in which water is used as a solvent, Example 9 is a composition with a PG formulation in which propylene glycol (PG) is used as a solvent, and Example 10 is a composition with a G formulation in which glycerin (G) is used as a solvent.

**[Table 3]**

| | Solvent | Solvent content (% by mass) | Lixisenatide content (% by mass) | Polymeric carrier | Polymeric carrier content (% by mass) |
|---|---|---|---|---|---|
| Example 8 (Aqueous formulation) | Water | 50 | 37.5 | Pullulan | 12.5 |
| Example 9 (PG formulation) | Propylene glycol (PG) | 50 | 50 | - | - |
| Example 10 (G formulation) | Glycerin (G) | 50 | 50 | - | - |

Microneedles were coated with the aforementioned GLP-1 analogue compositions from the tip portion of the needle to a height of 100 µm, whereby the coated microneedle arrays were obtained.

With Example 9 with a PG formulation and Example 10 with a G formulation, GLP-1 analogue compositions with a favorable viscosity of around 10000 cps were successfully prepared by virtue of the solvent itself having the high viscosity in combination with the viscosity derived from lixisenatide. Meanwhile, also with Example 8, a GLP-1 analogue composition with a favorable viscosity was successfully prepared by virtue of the effect of pullulan, which was added as a tackifier.

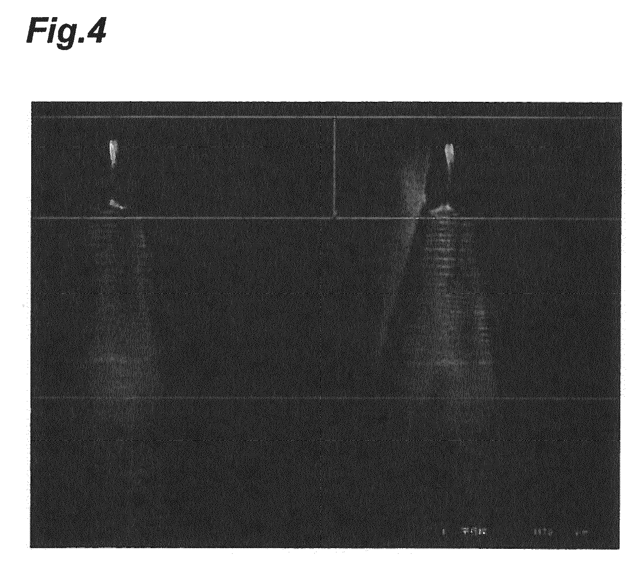

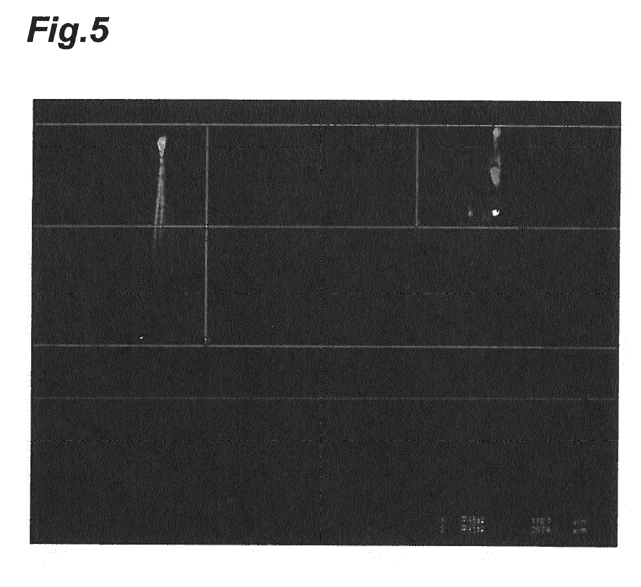

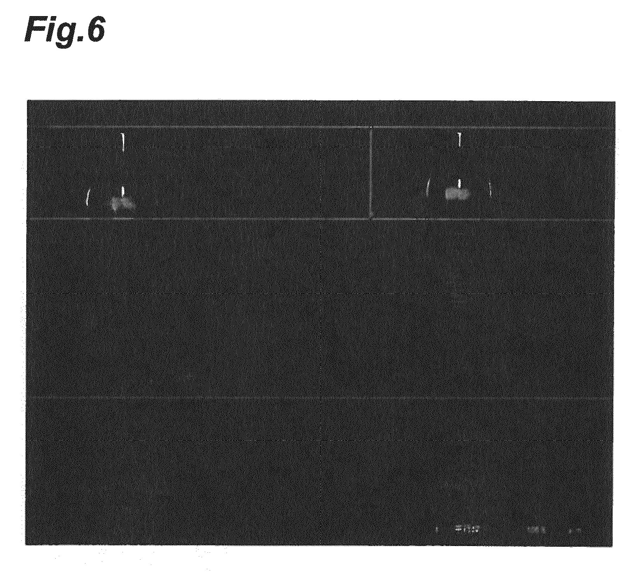

As a result of coating microneedles with the GLP-1 analogue compositions obtained in Examples 8 to 10, the intended lixisenatide content, which was 20 µg/patch, was successfully achieved. Figures 4, 5, and 6 are each photographs of the microneedles with the GLP-analogue compositions adhering thereto. Figure 4 shows the composition with an aqueous formulation (Example 8), Figure 5 shows the composition with a PG formulation (Example 9), and Figure 6 shows the composition with a G formulation (Example 10). The lixisenatide content was 48.7 µg in the aqueous formulation (Figure 4); 85.16 µg in the PG formulation (Figure 5), and 65.48 µg in the G formulation (Figure 6). Although the coating properties were favorable in all of these formulations, dripping was caused on some needles in the microneedle array with the PG formulation.

### <In vivo absorption test of lixisenatide in hairless rats>

### (Examples 11 to 13)

Microneedles were coated with the GLP-1 analogue compositions obtained in Examples 8 to 10, whereby microneedle arrays were obtained. The lixisenatide content in the coating on the microneedles was 7.96 µg/patch/head with the aqueous formulation; 12.2 µg/patch/head with the PG formulation; and 9.64 µg/patch/head with the G formulation. Using an impact applicator having each of the aforementioned microneedle arrays, lixisenatide was administered to hairless rats (repeated number of tests: three times).

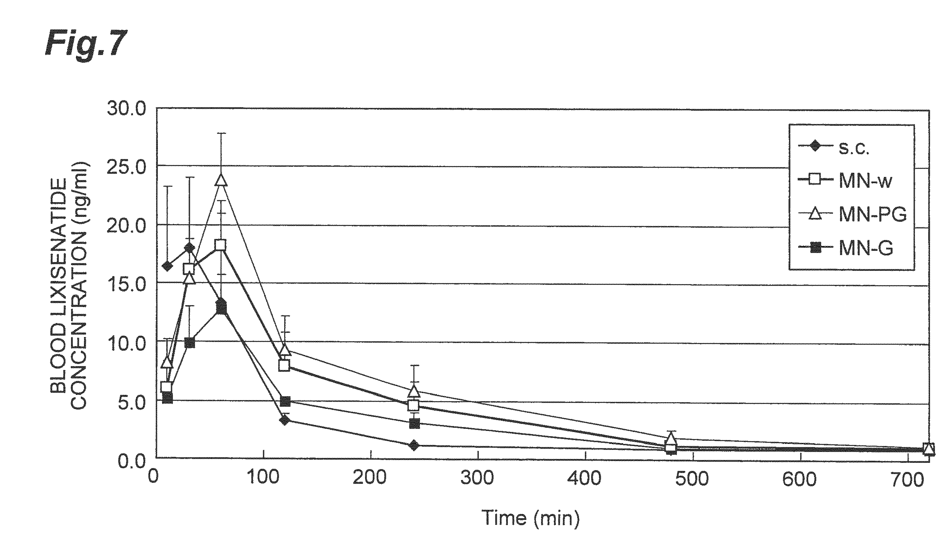

After 10 minutes, 30 minutes, 60 minutes, 120 minutes, 240 minutes, 480 minutes, and 720 minutes of the administration, 300 µL of blood was drawn from the jugular vein. Using the Exendin-4 EIA Kit, the blood lixisenatide concentration was measured. The results of measurement are shown in Figure 7. In Figure 7, "MN-w" indicates the microneedles with the aqueous formulation (Example 11), "MN-PG" indicates the microneedles with the PG formulation, and "MN-G" indicates the microneedles with the G formulation. The AUC values (area under the blood concentration-time curve) and the BA values (bioavailability) obtained from the graph of Figure 7 are shown in Table 4. The AUC value refers to the area under the blood concentration-time curve in the range from 0 minute to 720 minutes after the administration in the graph of Figure 7. The BA value refers to the relative bioavailability value with respect to the subcutaneous administration.

### (Comparative Example 1)

In a tube, lixisenatide and physiological saline were added at a mass ratio of 50:50, followed by mixing with a mixer. The resulting mixture was subcutaneously administered to a hairless rat under the condition of 15.1 µg/300 µL/head (repeated number of tests: three times). Subsequently, in a similar manner to Examples 11 to 13, the blood lixisenatide concentration was measured. The results of measurement are shown in Figure 7. In Figure 7, "s.c" refers to subcutaneous administration. The AUC and BA values are shown in Table 4.

**[Table 4]**

| | AUC value (ng·min/mL) | BA value (%, Based on subcutaneous administration) |
|---|---|---|
| Comparative Example 1 | 1422 | 100 |
| Subcutaneous administration (s.c.) | | |
| Example 11 | 3260 | 435 |
| Microneedles with aqueous formulation (MN-w) | | |
| Example 12 | 4079 | 355 |
| Microneedles with PG formulation (MN-PG) | | |
| Example 13 | 2266 | 250 |
| Microneedles with G formulation (MN-G) | | |

All of the microneedles with the above formulations exhibited higher BA values than that obtained by subcutaneous administration, whereby it was confirmed that an excellent blood profile of active ingredient was successfully obtained.

### <Long-term stability of the lixisenatide content>

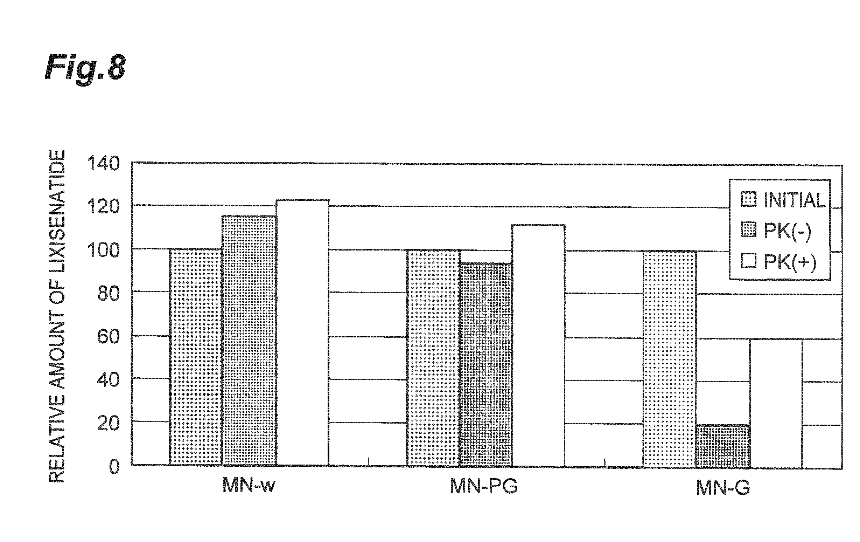

Microneedle arrays of Example 11 (aqueous formulation, MN-w), Example 12 (PG formulation, MN-PG), and Example 13 (G formulation, MN-G) each were left to stand under the environment of 60°C for one month. The microneedle arrays were left to stand while applying deoxidation treatment with an oxygen scavenger (PK(+)) or without applying deoxidation treatment (PK(-)). Figure 8 is a graph showing the lixisenatide content after leaving to stand as a relative value with respect to the initial value. With the aqueous formulation and PG formulation, a stable lixisenatide content was maintained even after leaving to stand. In the case of the G formulation, adsorption of lixisenatide to microneedles was facilitated, and as a result, the amount of lixisenatide to be extracted was reduced. However, it was confirmed that there was a tendency that applying deoxidation treatment improved the stability.

### <Discussion>

### Aqueous formulation

The composition with an aqueous formulation is absolutely excellent in all of the solubility, coating properties, blood profile, and stability. However, it is desirable to add a polymeric carrier to the composition in order to obtain a viscosity suitable for coating microneedles. As the polymeric carrier, a polymeric carrier capable of increasing viscosity at low concentration ( pullulan) is desirable also to prevent a decrease in the lixisenatide concentration.

Polyhydric alcohol formulation (not claimed) As a solvent in which a drug is dissolved or dispersed, not only water, but also a polyhydric alcohol is occasionally selected. Particularly, since glycerin and propylene glycol are higher in viscosity than water, when these substances are used as a solvent, a composition having a suitable viscosity for coating is easily formed even without using a polymeric carrier. Particularly, in the case of a drug of which solubility in a polyhydric alcohol is high (for example, a saturation concentration of 30% by mass or more), a polyhydric alcohol is suitable. According to propylene glycol and glycerin, which were used in Examples, the solubility and blood prolife are both favorable. With regard to the coating properties on microneedles, the glycerin formulation was favorable, while the propylene glycol formulation showed a tendency to cause dripping. However, given that the PG formulation has shown a favorable blood concentration profile as well as a high bioavailability value as a result of the in vivo administration test, the aforementioned drawback is assumed not to be a particular problem. It is assumed that the coating properties of the PG formulation still can be fully improved by a method such as further increasing the drug concentration and adding a viscosity improver that is highly compatible with PG.

### Reference Signs List

1: Microneedle device, 2: Microneedle substrate, 3: Microneedle, 5: GLP-1 analogue composition adhering to microneedle, and 10: GLP-1 analogue composition.

## Claims

1. A GLP-1 analogue composition for a microneedle device comprising:
a GLP-1 analogue;
water; and
a polymeric carrier, wherein the polymeric carrier is pullulan.

2. The GLP-1 analogue composition for a microneedle device according to claim 1, wherein the GLP-1 analogue is lixisenatide.

3. The GLP-1 analogue composition for a microneedle device according to any one of claims 1 or 2, wherein the GLP-1 analogue composition is adhered to a microneedle.

4. A production method of a microneedle device comprising:
having the GLP-1 analogue composition according to any one of claim 1 to 3 adhered on a microneedle

## Patentansprüche

1. GLP-1-Analogon-Zusammensetzung für eine Mikronadelvorrichtung, umfassend:
ein GLP-1-Analogon;
Wasser; und
einen Polymerträger, wobei der Polymerträger Pullulan ist.

2. GLP-1-Analogon-Zusammensetzung für eine Mikronadelvorrichtung nach Anspruch 1, wobei das GLP-1-Analogon Lixisenatid ist.

3. GLP-1-Analogon-Zusammensetzung für eine Mikronadelvorrichtung nach einem der Ansprüche 1 oder 2, wobei die GLP-1-Analogon-Zusammensetzung an eine Mikronadel gehaftet wird.

4. Herstellungsverfahren für einen Mikronadelvorrichtung, umfassend:
Bewirken, dass die GLP-1-Analogon-Zusammensetzung nach einem der Ansprüche 1 bis 3 an einer Mikronadel haftet.

## Revendications

1. Composition d'un analogue du GLP-1 pour un dispositif à micro-aiguille, comprenant :
un analogue du GLP-1 ;
de l'eau ; et
un porteur polymère, dans laquelle le porteur polymère est le pullulane.

2. Composition d'un analogue du GLP-1 pour un dispositif à micro-aiguille selon la revendication 1, dans laquelle l'analogue du GLP-1 est le lixisénatide.

3. Composition d'un analogue du GLP-1 pour un dispositif à micro-aiguille selon l'une quelconque des revendications 1 et 2, dans laquelle la composition d'un analogue du GLP-1 est adhérée à une micro-aiguille.

4. Procédé de production d'un dispositif à micro-aiguille comprenant :
l'adhésion de la composition d'un analogue du GLP-1 selon l'une quelconque des revendications 1 à 3 sur une micro-aiguille.
